# EUROPEAN PATENT APPLICATION

(11) **EP 3 637 094 A1**
(43) Date of publication of application: **15.04.2020**
(21) Application number: 17913892.0
(22) Date of filing: 15.06.2017
(51) Int. Cl.: G01N 24/08, G01R 33/46, G01R 33/56

(54) **MAGNETIC RESONANCE SPECTROSCOPY INTERACTION METHOD AND SYSTEM, AND COMPUTER READABLE STORAGE MEDIUM**

(71) Applicant: Shanghai United Imaging Healthcare Co., Ltd., Shanghai 201807 (CN)
(72) Inventor: WANG, Yanyan, Shanghai 201807 (CN); MA, Qingzhen, Shanghai 201807 (CN)
(74) Representative: Wang, Bo
(86) International application number: PCT/CN2017/088425
(87) International publication number: WO 2018/227469

(57) **Abstract**

The present disclosure relates to methods and systems for interaction in magnetic resonance spectroscopy. After two or more metabolites and one or more operators are selected based on an input of a user to generate a composite, a concentration and a spatial distribution of the composite may be calculated. And a calculation result including the concentration of the composite may be displayed to the user.

## Description

### TECHNICAL FIELD

The present disclosure relates to methods and systems for processing a magnetic resonance spectrum, in particular, relates to methods and systems for analysis and interaction in magnetic resonance spectroscopy.

### BACKGROUND

Magnetic resonance spectroscopy is a method for quantitative analysis of specific atomic nucleus and compounds using magnetic resonance phenomena and chemical shift effect. As a non-invasive technique for measuring the metabolism of compounds developed in recent years, the magnetic resonance spectroscopy may be used to detect the anomaly regarding certain tissues by measuring the concentration of a compound or a composite.

In the analysis of a magnetic resonance spectrum, the interaction for analyzing a composite may have some disadvantages including unfriendly user interface, limited user operations available, inflexible composite editing, etc., which cause poor user experience in diagnosis and reduce the diagnosis efficiency. Therefore, it is desirable to provide interactive methods and systems that are user friendly, clear, allow preview of edited compounds and flexible editing of composites to meet user's needs for performing diagnosis.

### SUMMARY

Additional features will be set forth in part in the description that follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

Some embodiments of the present disclosure provide a system for magnetic resonance spectroscopy. The system may include a computer-readable storage medium configured to store executable modules. The executable modules may include an editing module configured to select, based on an input of a user, at least two metabolites and one or more operators to generate a composite; a calculation module configured to calculate a concentration of the composite; a display module configured to display to the user a calculation result including the concentration of the composite. The system may also include a processor configured to execute the executable module stored in the computer-readable storage medium.

In some embodiments, the editing module may include a metabolite list unit configured to provide a plurality of metabolites for selection.

In some embodiments, the editing modules may further include an operator unit configured to provide a plurality of operators for selection.

In some embodiments, the editing modules may further include a preview unit configured to display one or more compounds and the one or more operators.

In some embodiments, the calculation module may be to configured to receive the compound from the editing module and calculate the concentration of the composite.

In some embodiments, the calculation module may be configured to analyze the composite using a reverse polish notation algorithm to obtain the at least two metabolites and the one or more operators and calculate the concentration of the composite based on a concentration of each of the at least two metabolites and the one or more operators.

In some embodiments, the display module may be configured to display a spatial distribution of the concentration of the composite in an object.

Some embodiments of the present disclosure disclose a method for analyzing a magnetic resonance spectrum. The method may include selecting, based on an input of a user, at least two metabolites and one or more operators to generate a composite; calculating a concentration of the composite; and displaying a calculation result including the concentration of the composite.

In some embodiments, the method may further include displaying the at least two metabolites and the one or more operators.

In some embodiments, the calculating the concentration of the compound may further include analyzing the composite using a reverse polish notation algorithm to obtain the at least two metabolites and the one or more operators and calculating the concentration of the composite based on a concentration of each of the at least two metabolites and the one or more operators.

In some embodiments, the displaying the calculation result may including the concentration of the composite may further include displaying a spatial distribution of the concentration of the composite in an object.

Some embodiments of the present disclosure disclose a computer-readable storage medium configured to store an executable program to perform following operations including selecting, based on an input of a user, at least two metabolites and one or more operators to generate a composite; calculating a concentration of the composite; displaying to the user a calculation result including the concentration of the composite; and a processor configured to execute the executable program stored in the computer-readable storage medium.

In some embodiments, the executable program may further perform an operation of displaying the at least two metabolites and the one or more operators.

In some embodiments, the executable program may further perform the following operations including calculating the concentration of the compound includes analyzing the composite using a reverse polish notation algorithm to obtain the at least two metabolites and the one or more operators and calculating the concentration of the composite based on a concentration of each of the at least two metabolites and the one or more operators.

In some embodiments, the displaying the calculation result including the concentration of the compound may further include displaying a spatial distribution of the concentration of the compound in a body of an object.

### BRIEF DESCRIPTION OF THE DRAWINGS

In order to illustrate the technical solutions related to the embodiments of the present disclosure, brief descriptions of the drawings referred to the description of the embodiments is provided below. Obviously, the drawings in the following description are only some examples of the present disclosure. For those skilled in the art, the present disclosure may be applied to other similar scenarios according to these drawings without any creative effort. Unless stated otherwise or obvious from the context, reference numerals represent similar structures throughout the several views of the drawings and operations.
FIG. 1A is a schematic diagram illustrating a system for magnetic resonance spectrum processing according to some embodiments of the present disclosure;
FIG. 1B is an architectural diagram illustrating a computer device on which the compound editing and interaction system(s) 130 may be implemented according to some embodiments of the present disclosure.;
FIG. 2A illustrates a spectrum signal in a time domain collected by a spectrum signal acquisition device;
FIG. 2B illustrates a spectrum signal in a frequency domain obtained by Fourier transform;
FIG. 3 is a schematic diagram illustrating an exemplary system for interactive compound editing according to some embodiments of the present disclosure;
FIG. 4 is a schematic diagram illustrating an editing module according to some embodiments of the present disclosure;
FIG. 5 is a flowchart illustrating an exemplary process for displaying a concentration of a generated composite to a user according to some embodiments of the present disclosure ;
FIG. 6 is a flowchart illustrating a process for magnetic resonance spectroscopy analysis and interaction regarding an editable composite according to some embodiments of the present disclosure; and
FIG. 7 illustrates an image of the spatial distribution of a composite concentration obtained according to the process in FIG. 6.

### DETAILED DESCRIPTION

In order to illustrate the technical solutions related to the embodiments of the present disclosure, brief introduction of the drawings referred to in the description of the embodiments is provided below. Obviously, drawings described below are only some examples or embodiments of the present disclosure. Those having ordinary skills in the art, without further creative efforts, may apply the present disclosure to other similar scenarios according to these drawings. Unless stated otherwise or obvious from the context, the same reference numeral in the drawings refers to the same structure and operation.

As used in the disclosure and the appended claims, the singular forms "a," "an," and "the" include plural referents unless the content clearly dictates otherwise. In general, the terms "comprise" and "include" merely prompt to include steps and elements that have been clearly identified, and these steps and elements do not constitute an exclusive listing. The methods or the devices may also include other steps or elements.

The present disclosure may include some references to some modules in some the embodiments of the system in the present disclosure. However, a different number of modules may be used and run on the client and/or server. These modules may only be used for illustration purposes, and different modules may be used in different aspects of the system and method.

The flowcharts used in the present disclosure may illustrate operations that systems implement according to some embodiments in the present disclosure. It should be understood that the preceding or following operations may not be necessarily performed exactly in order. Conversely, the operations may be performed in inverted order, or simultaneously. Besides, one or more other operations may be added to the flow charts, or one or more operations may be omitted from the flow chart.

The "scanning region" may represent an actual region where a scan is performed. In some embodiments, a region of interest of an object may be scanned. The region of interest may be the whole or a part of the object, for example, the head, the chest, the abdomen, the heart, a liver, an upper limb, a lower limb, the spine, a bone, a blood vessel, a lesion, a tumor, etc., or any combination thereof. In some embodiments, scanned images of a selected slice of the region of interest may be collected at a plurality of time points.

The "element" may represent the smallest component in an image matrix, and the "voxel" may represent the smallest component in an actual region. Unless the context clearly indicates an exception, the "element" in the image matrix and the "voxel" in the actual region corresponding to the image matrix in the present disclosure may have the same meaning and may be used interchangeably.

An aspect of the present disclosure relates to an analysis and interaction system for magnetic resonance spectroscopy. The analysis and interaction system for magnetic resonance spectroscopy may include an editing module, a calculation module, and a display module. Another aspect of the present disclosure relates to a method for displaying a concentration distribution of an edited composite or a change of the concentration distribution. The method for interaction in magnetic resonance spectroscopy described in the present disclosure may include performing a concentration calculation and a visual display of a composite that needs to be edited and generated based on an input of a user.

Some embodiments of the present disclosure may be applied to different image processing systems. Such different image processing systems may include a magnetic resonance imaging (MRI) system, a magnetic resonance imaging-positron emission tomography system (MR-PET system), etc.

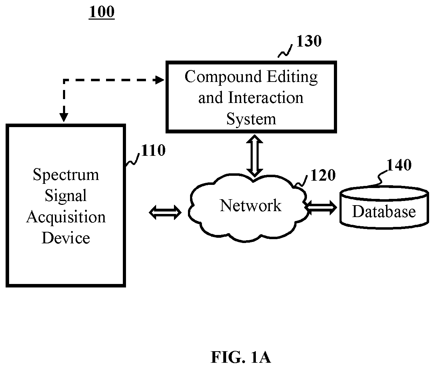

FIG. 1A is a schematic diagram illustrating a system for magnetic resonance spectroscopy according to some embodiments of the present disclosure. Magnetic resonance spectroscopy (MRS), as a technique for non-invasive detection of biochemical characteristics of living tissue, may be used to study pathological and physiological changes of human cell metabolism. In many diseases, a metabolic change may precede a pathological change. Moreover, MRS may be highly sensitive to a metabolic change, so it may provide information for early detection of lesions. The magnetic resonance spectroscopy system 100 may include one or more spectrum signal acquisition devices 110, one or more networks 120, one or more compound editing and interaction systems 130, and one or more databases 140.

The spectrum signal acquisition device(s) 110 may scan an object to obtain scan data. The scan data may be sent to the compound editing and interaction system(s) 130 via the network(s) 120 for further processing or may be directly stored in the database(s) 140 via the network(s) 120. In some embodiments, the spectrum signal acquisition device(s) 110 may be directly connected to the compound editing and interaction system(s) 130. The scan data may be sent directly to the compound editing and interaction system(s) 130. The object may include a human body, an animal, or the like. The spectrum signal acquisition device(s) 110 may include, but is not limited to, a magnetic resonance imaging (MRI) device, a magnetic resonance imaging-positron emission tomography hybrid system (MR-PET system), or the like.

The compound editing and interaction system(s) 130 may process and analyze input data (e.g., spectrum signals collected by the spectrum signal acquisition device(s) 110 and/or the spectrum signals stored in the database(s) 140, one or more operators selected by a user, etc.) to generate a processing result. For example, the compound editing and interaction system(s) 130 may generate a composite based on a plurality of selected metabolites. As another example, the compound editing and interaction system(s) 130 may visualize concentration data of the composite to generate a spatial distribution image of the concentration of the composite. The scanned image may be a two-dimensional image or a three-dimensional image. The compound editing and interaction system(s) 130 may include a processor and an input/output apparatus (not shown). In some embodiments, the processor may be a server or a server group. The server group may be centralized, for example, a data center. The server group may be distributed, for example, a distributed system. The processor may be a combination of one or more of a cloud server, a file server, a database server, a file transfer protocol (FTP) server, an application server, a proxy server, a mail server, or the like. The processor may be a local processor or a remote processor. The local processor may include a processor integrated in the compound editing and interaction system(s) 130. The remote processor may include a processor coupled to the compound editing and interaction system(s) 130 via a network (e.g., the network(s) 120). In some embodiments, the processor may access information stored in the database(s) 140 (e.g., a medical image stored in the database(s) 140), information in the spectrum signal acquisition device(s) 110 (e.g., a medical image taken by the spectrum signal acquisition device(s) 110). In some embodiments, the input/output apparatus may input data to the processor, and may receive data output by the processor, and display the output data in the form of numbers, characters, images, videos, animations, sounds, or the like. In some embodiments, the input/output apparatus may include, but is not limited to, an input apparatus, an output apparatus, or the like, or any combination thereof. The input apparatus may include, but is not limited to, a character input apparatus (e.g., a keyboard), an optical reading device (e.g., an optical mark reader, an optical character reader), a graphic input apparatus (e.g., a mouse, a joystick, a light pen), an image input apparatus (e.g., a camera, a scanner, a fax machine), an analog input apparatus (e.g., a language analog to digital conversion recognition system), or the like, or any combination thereof. The output apparatus may include, but is not limited to, a display apparatus, a printing apparatus, a plotter, a video output apparatus, a speech output apparatus, a magnetic recording apparatus, or the like, or any combination thereof. In some embodiments, the compound editing and interaction system(s) 130 may further include a storage apparatus (not shown). The storage apparatus may store various information, for example, programs, data, or the like. In some embodiments, the data and/or processing results generated by the compound editing and interaction system(s) 130 (e.g., an anatomical image, a spectrum signal in the frequency domain, an spatial distribution image of the concentration, etc.) may be stored in the storage apparatus of the database(s) 140 and/or the compound editing and interaction system(s) 130, and may also be output via the input/output apparatus.

The database(s) 140 may include a device that may provide a storage function. The database(s) 140 may store scan data collected from the spectrum signal acquisition device(s) 110 and various data generated in the operation of the compound editing and interaction system(s) 130. The database(s) 140 may be a local database or a remote database. The local database may include a device with the storage function integrated in the database(s) 140. The remote database may include a device with the storage function connected to the database(s) 140 via a network (e.g., the network(s) 120). The database(s) 140 may include, but is not limit to, a hierarchical database, a network database, a relational database, or the like, or any combination thereof. The database(s) 140 may digitize information, and then store the digitized information in the storage device by an electrical method, a magnetic method, an optical method, or the like. The database(s) 140 may be used to store various information, for example, software, data, or the like. The database(s) 140 may be a device that stores information by means of electricity methods, for example, various storages, a random-access memory (RAM), a read-only memory (ROM), or the like. The RAM may include, but is not limit to, a decade counting tube, a selectron storage, a delay line memory, a Williams tube, a dynamic random access memory (DRAM), a static random access memory (SRAM), a thyristor random access memory (T-RAM), a Zero capacitor RAM (Z-RAM), or the like, or any combination thereof. The ROM may include, but is not limit to, a magnetic bubble memory, a magnetic button line memory, a film memory, a magnetic plated wire memory, a magnetic core memory, a magnetic drum memory, an optical drive, hard disk, a magnetic tape, an early nonvolatile random access memory (NVRAM), a phase change memory, a magnetoresistive random access memory, a ferroelectric random access memory, a non-volatile SRAM, flash memory, an electronically erasable read only memory, an erasable programmable read only memory, a programmable read-only memory, a masked heap read memory, a floating connection gate random access memory, a nano random access memory, a racetrack memory, variable resistance memory, a programmable metallization unit, or the like, or any combination thereof. The database(s) 140 may be a device that stores information by means of magnetic energy methods, for example, a hard dick, a soft disk, a tape, a magnetic core storage, a magnetic bubble memory, a U flash disk, or the like, or a combination thereof. The database(s) 140 may be a device that stores information by means of optical manners, for example, a CD, a DVD, or the like. The database(s) 140 may be a device that stores information by means of magneto-optical manners, for example, a magneto-optical disk, or the like. Access modes of the database(s) 140 may include a random access mode, a serial access mode, a read-only access mode, or the like, or any combination thereof. The database(s) 140 may be a non-permanent memory or a permanent memory. The storage devices mentioned above are just a few examples, and the database that may be used in the magnetic resonance spectroscopy system 100 is not limited to these.

The network(s) 120 may be a single network or a combination of multiple networks. The network(s) 120 may include, but is not limited to, a local area network, a wide area network, a public network, a dedicated network, a wireless local area network, a virtual network, a metropolitan area network, a public switched telephone network, or the like, or any combination thereof. The network(s) 120 may include various network access points, for example, wired or wireless access points, base station or network switch points, by which data source may be connected with the network(s) 120 and the information may be transmitted via the network.

It should be noted that the above description of the system for analyzing the magnetic resonance spectrum is merely an example, and the present disclosure may not be limited to the scope of the embodiments. For persons having ordinary skills in the art, modules may be combined in various ways, or connected with other modules as sub-systems. Various variations and modifications may be conducted under the teaching of the present disclosure. However, the variations and the modifications may not depart the spirit and scope of this disclosure. For example, in some embodiments, the database(s) 140 may be a cloud computing platform with a data storage function, including, but is not limited to, public clouds, private clouds, community clouds, hybrid clouds, or the like. All such variations are within the protection scope of the present disclosure.

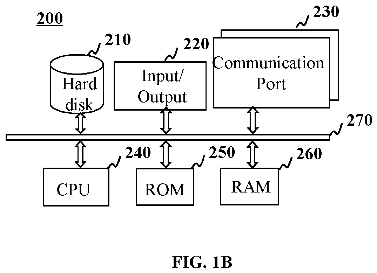

FIG. 1B is an architectural diagram illustrating a computer device on which the compound editing and interaction system 130 may be implemented according to some embodiments of the present disclosure. The computer may be a general purpose computer or a specific purpose computer. The compound editing and interaction system(s) 130 may be implemented by the computer device through its hardware devices, software programs, firmware, or any combinations thereof. For convenience, only one computer device is shown in FIG. 1B, but the related functions of the system for the spectrum signal acquisition device(s) 110 may be implemented by a plurality of computer devices in a distributed manner.

The computer device may include a communication port 230, a network (for example, the network(s) 120 in FIG. 1A) for implementing data communication may be connected to the communication port 230. The computer device architecture may also include a central processing unit (CPU) 240 for executing program commands, consisting of one or more processors. The computer device architecture may include an internal communication bus 270, different forms of program storage units and data storage units, for example, a hard disk 210, a read only memory (ROM) 250, a random access memory (RAM) 260, which may be configured to store various data files used by the computer device architecture for processing and/or communicating, and possible program commands executed by the CPU unit 240. The computer device architecture may also include an input/output component 220 that supports data and/or information interaction between the computer device architecture and an external (for example, the user). The computer device architecture may also receive programs or data via a communication network.

The magnetic resonance spectroscopy may provide metabolic information of a tissue. The tissue may be an organ, a body fluid, nerves, cells, or the like, or any combination thereof, of a human or an animal. The magnetic resonance spectroscopy may operate based on the difference in precession frequencies of magnetic nucleus of a same type caused by different structures of different molecules. Specifically, as the magnetogyric ratio of a magnetic nucleus is constant, in addition to the influence of an applied static magnetic field, the magnetic nucleus in an external magnetic field of an intensity may be influenced by an electron cloud around the magnetic nucleus and electron clouds of other atoms around it, which cause the magnetic field intensity sensed by the magnetic nucleus slightly lower than the magnetic field intensity of the applied static magnetic field, thereby resulting in a decreased precession frequency of the magnetic nucleus. If magnetic nuclei of the same type are in different molecules, due to the difference in the chemical structures of the molecules, the magnetic shielding effect of electron clouds on the magnetic nuclei may also be different, which may be reflected in the difference between the precession frequencies of the magnetic nuclei of the same type in different molecules. The phenomenon of the difference in the precession frequencies of magnetic nuclei of the same type in different molecules caused by the different chemical structures of the molecules is called the chemical shift effect. Taking the H proton as an example, a designed radio-frequency pulse may be applied to a target region, and the frequency range of the designed radio-frequency pulse may cover the precession frequencies of protons in one or more metabolites to be detected. And then an MR signal of the target region (e.g., an FID signal or an echo signal) may be collected. The MR signal may be derived from protons in metabolites of various types. Due to the chemical shift effect, the precession frequencies of protons in different metabolites may be slightly different, and the information of spectral lines of different substances may be obtained by Fourier transform. The spectral lines may include a series of relatively narrow peaks. The abscissa of the spectral lines may indicate the precession frequencies of protons in different substances, usually denoted by parts per million (PPM) (which is based on the precession frequency of protons in a standard substance and denotes the difference between the precession frequency of protons in a metabolite and the precession frequency of protons in the standard substance). An area under the peak of a narrow wave may be proportional to the content or the concentration of a metabolite of a specific type in the target region. Therefore, the concentration of the metabolite may be calculated. More descriptions may be found in relevant paragraphs below. For example, ¹H, ³¹P, ¹²C, ²³Na, and ¹⁹F may be collected for magnetic resonance spectroscopy, which may be used for diagnosis and differential diagnosis of a brain tumor, a cerebral ischemic disease, a prostate cancer, a diffuse liver disease, a renal function analysis, a renal transplant rejection, a brain change in a metabolic disease, a recurrence brain tumor after a treatment, etc. In some embodiments, a relatively stable chemical substance may be determined as a standard substance for the precession frequency of a metabolite associated with a magnetic nucleus. For example, the trimethylsilane may be used as the standard substance for ¹H MRS, and the creatine phosphate may be used as the standard substance for ³¹P MRS. In some embodiments, because the concentration of a metabolite is low, the generated MR signal associated with the metabolite may be almost one ten-thousandth of the normal signal of water. Therefore, the sensitivity of MRS is relatively low, and MR signals may be obtained based on water inhibition and fat suppression.

Metabolites in ¹H magnetic resonance spectroscopy of the brain may include (1) NAA (N-acetyl aspartate), which mainly exists in neurons and axons and may be used as an internal standard of the neurons, whose content may reflect the function state of the neurons, and the decrease of the content may indicate neuronal damage; (2) Creatine (Cr), an energy metabolite, whose concentration may be relatively stable in brain tissues, and generally used as an internal standard of ¹H MRS in the brain tissues. The ratios of other metabolites to Cr may reflect changes of other metabolites; (3) Choline (CHo), which mainly exists in the cell membrane. The change of the content of CHo may indicate a change in cell membrane metabolism, and the content of CHo may increase when the cell membrane is degraded or synthesized. The content of Cho may increase and the content of NAA may decrease when there is a tumor in the brain, thereby causing an increase in the ratio of Cho to NAA., especially in malignant tumors. Demyelinating lesions such as multiple sclerosis may exist lesion activities if the Cho is elevated; (4) Lactic acid (Lac), an end-product of glycolysis. In some embodiments, ¹H MRS may not show an obvious Lac peak, but if there is cerebral ischemia or a malignant tumor in the brain, the anaerobic glycolysis of carbohydrates may be strengthened, and the Lac content may increase.

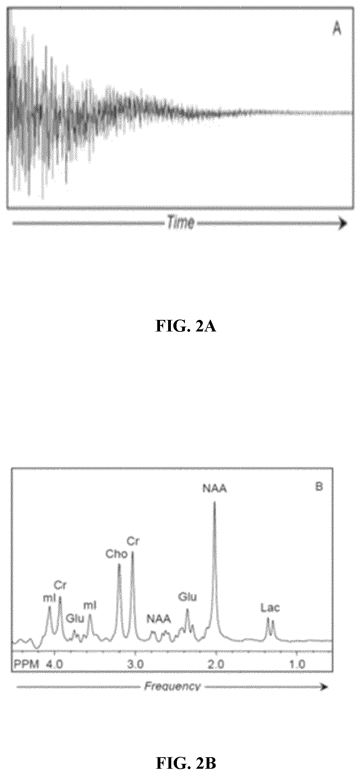

In some embodiments, a collected spectrum signal associated with a metabolite in the time domain may be converted into a signal in the frequency domain based on the Fourier transform (FFT transform), and then the metabolite concentration may be calculated by operations including, for example, a baseline correction, a phase correction, a curve fitting, or the like, to fit the metabolite. For example, FIG. 2A illustrates a spectrum signal in a time domain collected by the spectrum signal acquisition device(s) 110. FIG. 2B illustrates a spectrum signal in a frequency domain. NAA, Cr, Cho, and metabolites or compounds of other types may be fitted at different PPMs after performing the operations.

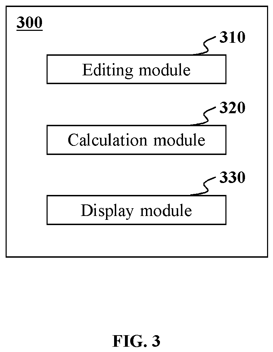

FIG. 3 is a schematic diagram illustrating an exemplary compound editing and interaction system according to some embodiments of the present disclosure. The compound editing and interaction system 300 may include, but is not limited to, an editing module 310, a calculation module 320, and a display module 330. The editing module 310, the calculation module 320, and the display module 330 may be implemented by the CPU 240 shown in FIG. 2.

The editing module 310 may edit compounds or metabolites in a list to generate a composite that the user needs to view. The metabolites in the list may be derived from intermediates or compounds generated in magnetic resonance spectroscopy using specific chemical substances including, but not limited to, ¹H, ³¹P, ¹²C, ²³Na, and ¹⁹F. In some embodiments, the metabolites in the list may relate to a person involved in the magnetic resonance spectroscopy. For example, the metabolites in the list may only display metabolites associated with the magnetic resonance spectroscopy of a patient with a brain tumor according to his identity. In some embodiments, the list may intelligently suggest one or more composites of which the metabolites may be composed. For example, when a user clicks or highlights a location corresponding to metabolite A in the list, the compound editing and interaction system(s) 130 may provide a pop-up window displaying a group of candidate composites, such as B1, B2, ... , etc., each of which includes metabolite A and one or more other metabolites. In some embodiments, the editing module may provide various operators for editing the metabolites listed in the list. For example, the operators may include an addition operator, a subtraction operator, a multiplication operator, a division operator, left and right parentheses, a deletion operator, and a confirmation operator. The editing module may also provide a preview function for a composite that is scheduled to be edited. For example, the editing module may display the selected metabolites and operators in real time, and/or display one or more composites that may be expected to be generated. In some embodiments, the editing module may display one or more composites to be edited in a planar way or a stereoscopic way. For example, the editing module may display a generated composite by animation, in which atoms and/or molecules in the metabolites of the generated composite may be dynamically simulated using spheres of different colors and radii.

The calculation module 320 may calculate the spatial concentration of a composite generated by the editing module in an inputted magnetic resonance (MR) anatomical image based on the spectrum signal of metabolites in the time domain related to the composite transmitted by the spectrum signal acquisition device(s) 110. The MR anatomical image and the spectrum signal information in the time domain may include information of the object simultaneously obtained. For example, a single slice and multi-voxel spectrum with size 18*14 obtained using chemical shift imaging (i.e., CSI data) may include 252 voxels. The calculation module may generate an 18*14 concentration matrix of a composite by calculating the concentration of the composite in each voxel. The concentration matrix of the composite may be displayed by certain visualization techniques. For example, the positions of voxels in the CSI data displayed on the anatomical image may be determined first based on the spatial positional relationship of the CSI data and the anatomical image. For example, the CSI data may be denoted as a cuboid in a three-dimensional space that may be identified by vectors of a vertex and three edges of the cuboid. The CSI data may be represented in a two-dimensional image in the three-dimensional space by calculating the intersection slice of the CSI with the two-dimensional image.

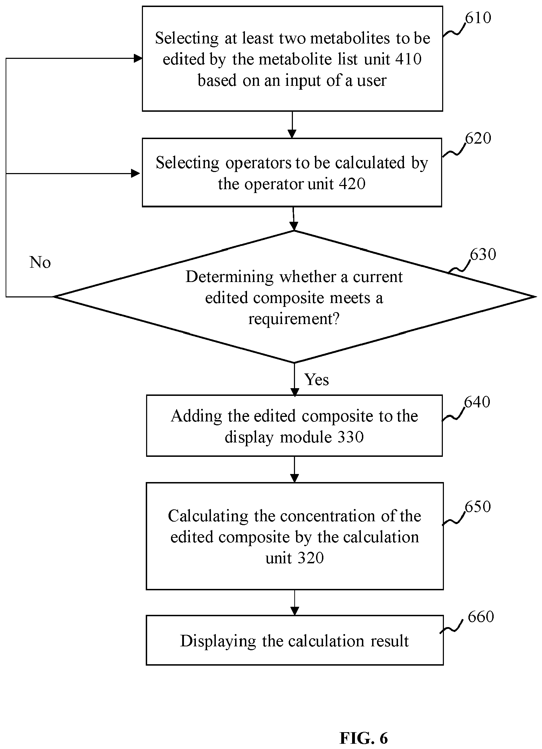

If a field of view (FOV) and a volume of interest (VOI) are determined, the calculation unit may perform an interpolation operation on the 18*14 concentration matrix to obtain an interpolated concentration matrix with the same size of the FOV and fuse the interpolated concentration matrix with the anatomical image. The fusion of the interpolated concentration matrix and the anatomical image may refer to that displayed values in a fused image may be calculated according to a certain fusion ratio based on a reference image and a concentration color graphic. For example, if a pixel value of a certain point in the reference image is ImageValue, a value corresponding to the certain point in the concentration color graphic is MetaboliteValue, and the fusion ratio is a factor, an actual displayed value corresponding to the certain point may be ActualValue = ImageValue*(1-factor)+MetaboliteValue*factor. More descriptions for fusion may be found in FIG. 6 and the descriptions thereof.

The display module 330 may be used to visualize the calculation result of a composite and display the spatial change of the concentration of the composite. For example, the calculation module may transmit the fusion result of the calculated concentration matrix and the anatomical image to the display module. The display module may display the spatial distribution of the concentration in the region of interest. In some embodiments, the change of the displayed spatial distribution of the concentration with time may also be displayed by the display module. For example, after the calculation module calculates and fuses concentration matrixes in the region of interest by inputting a series of anatomical images with time stamps, the series of fused anatomical images may display the change of the spatial distribution of the concentration with time.

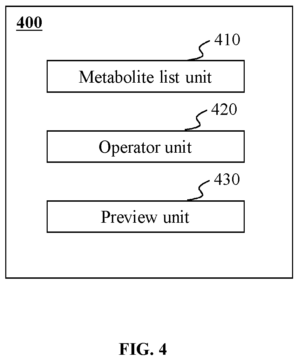

FIG. 4 is a schematic diagram illustrating an exemplary editing module according to some embodiments of the present disclosure. The editing module 400 may include, but is not limited to, a metabolite list unit 410, an operator unit 420, and a preview unit 430. The metabolite list unit 410, the operator unit 420, and the preview unit 430 may be implemented by a computer device as shown in FIG. 1B via integral or partial components of the CPU 240.

The metabolite list unit 410 may display a list of metabolites involved in magnetic resonance spectroscopy. The metabolites in the list may be derived from intermediates or compounds generated by the magnetic resonance spectroscopy using specific chemical substances including, but not limited to, ¹H, ³¹P, ¹²C, ²³Na, and ¹⁹F (or the like). For example, the metabolites may include NAA, Cho, GLu, Cr, ml, Lac, etc. Further, the metabolites in the list may also include other compounds that may be involved in magnetic resonance spectroscopy of the brain. The update of the list may be in real-time or not. The list may be a simple list. For example, the metabolites in the list may be arranged in a row or a column. The list may be a complex list. For example, the list may be a drop-down list including sub-lists that may be expanded by clicking on intermediate nodes in the list. In some embodiments, the metabolites in the list may be related to a person involved in the magnetic resonance spectroscopy. For example, the metabolites in the list may only display metabolites associated with the magnetic resonance spectroscopy of an object according to his identity. The object mentioned here may be a person, an animal, an organ, a tissue, a body fluid, or the like, or any combination thereof.

The operator unit 420 may provide various operators for editing the metabolites listed in the list. For example, the operators may include an addition operator, a subtraction operator, a multiplication operator, a division operator, left and right parentheses, a deletion operator and a confirmation operator. The operators may also include a logarithm operator, an index operator, a derivation operator, an integration operator, a square operator, a cube operator, a fourth power operator, a square root operator, a cube root operator, and a fourth root operator. Further, the operator unit may have a programming function, and may edit a function by combining operators according to a user setting. For example, *f(x)* = *ax*∗*x* + *bx* + *c* may be a function that may be used to calculate the concentration of a composite.

In some embodiments, the metabolite list unit 410 and the operator unit 420 may also be integrated into one single unit. For example, the metabolite list unit 410 and the operator unit 420 may express a composite using a reverse polish notation algorithm. In a general expression, a binary operator may be placed between two operands associated with the binary operator (e.g., 1+1), which may also be referred to as an infix representation. In 1929, Polish logician J. Lukasiewicz proposed another algorithm for denoting expressions, called the reverse polish notation algorithm. All operators are placed after operands in the reverse polish notation algorithm, which may also be referred to as a suffix notation. For example, a formula *a* + *b* may be denoted as *a, b,* + in the reverse polish notation algorithm. The reverse polish notation algorithm may convert a complex expression into an expression that may be used to calculate a result through one or more simple operations. The reverse polish notation algorithm may use merely two simple operations (pushing and popping) to achieve a same calculation of a general expression. The pushing and the popping used herein are two standard operations associated with a stack operation in computer data structures.

The preview unit 430 may display selected metabolites and operators, and/or display a generated composite. The display may be real-time or not. In some embodiments, the editing module may display one or more composites to be edited in a planar manner or a stereoscopic manner. For example, the editing module may display a generated composite by animation, and in which atoms and/or the molecules in the metabolites of the generated composite may be dynamically simulated using spheres of different colors and radii. In some embodiments, the preview unit 430 may provide an information reminder smartly for the generated composite. For example, the preview unit 430 may indicate whether the generated composite is a compound that actually exists in the physical world. As another example, the preview unit 430 may display the relevant chemical properties of the generated composite, and/or the physiological or pathological information of the object involved, or the like.

The above description is only a specific embodiment of the present disclosure and should not be considered as the only embodiment. It will be apparent to those skilled in the art that various modifications and changes in form and detail may be made without departing from the principles and structure of the present disclosure. However, such modifications and changes are still within the scope of the claims of the present disclosure. For example, alternatively, the editing module 310 may include a composite editing and generation unit, showing all the composites generated by the metabolite list unit 310.

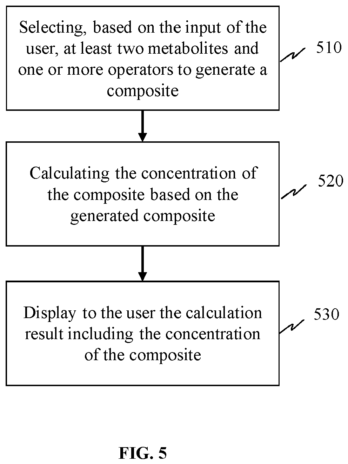

FIG. 5 is a flowchart illustrating an exemplary process for displaying a concentration of a composite to a user according to some embodiments of the present disclosure. In some embodiments, process for the display of the concentration of the composite may be implemented by a processor 200. As shown in FIG. 5, in 510, the processor 200 may select, based on an input of the user, at least two metabolites and one or more operators to generate a composite. In some embodiments, the input of the user may include the selected at least two metabolites and one or more operators. A composite may be expected to be generated based on the at least two metabolites and one or more operators selected by the user. In some embodiments, the input of the user may also include a spectrum signal corresponding to the selected at least two metabolites in a time domain. The spectrum signal in the time domain may be obtained by a scanning of an MRI, an MRI-PET, etc., or may be inputted by the spectrum signal acquisition device(s) 110. Alternatively, the spectrum signal in the time domain may also be obtained by other means. Further, the input of the user may also include the spectrum signal in a frequency domain generated by performing Fourier transform on the spectrum signal in the time domain. In some embodiments, spatial concentration distributions of the at least two metabolites selected by the user may also be inputted in operation 510. Operation 510 may be performed by the editing module 310.

In 520, the concentration of the generated composite may be determined based on the generated composite. The calculated concentration of the composite may include the spatial distribution of the concentration of the composite or the change of the spatial distribution of the concentration of the composite. For example, taking composite NAA/(Cr+Cho) as an example, compounds NAA, Cr and Cho and operators "/", "+", "("and ")" may be analyzed from the formula using the reverse polish notation algorithm. And then the concentration of the composite may be calculated based on the analysis result. For example, in a certain voxel, NAA has a concentration of 43, Cr has a concentration of 30, and Cho has a concentration of 47, then the concentration of composite NAA/(Cr+Cho) in the voxel may be 43/(30+47), which is approximately equal to 0.56. If the concentration of NAA, Cr, and Cho changes over time, the change of the concentration of composite NAA/(Cr+Cho) over time may be calculated in operation 520. In some embodiments, operation 520 may be performed by the calculation module 320.

In 530, the calculation result including the concentration of the composite may be displayed to a user. As described above, the calculated concentration of the composite may include the spatial distribution of the concentration of the composite or the change of the spatial distribution of the concentration of the composite with time. In some embodiments, the calculation result may be displayed using a visualization technique to display the spatial variations of the concentration. For example, a single slice and multi-voxel spectrum with size CSI 18*14 may include 252 voxels. The concentration matrix with size 18*14 of the composite may be generated by the calculation module 320. The positions of the CSI data displayed on the anatomical image and the size of the FOV and the VOI may be determined based on the spatial positional relationship between the CSI data and the anatomical image, and the 18*14 concentration matrix may be interpolated to obtain an interpolated concentration matrix with the same size of the FOV and fuse the interpolated concentration matrix with the anatomical image. And the change of the concentration may be only displayed in the VOI. In some embodiments, operation 530 may be implemented by the display module 330.

FIG. 6 is a flowchart illustrating a process for interaction in magnetic resonance spectroscopy of an editable composite according to some embodiments of the present disclosure.

In 610, the at least two metabolites may be selected to be edited by the metabolite list unit 410 based on an input of the user. Alternatively, the input of the user may also include a spectrum signal corresponding to the selected at least two metabolites in a time domain. The spectrum signal in the time domain may be obtained by a scanning by an MRI, an MRI-PET, etc., or may be input by the spectrum signal acquisition device 110. Alternatively, the spectrum signal in the time domain may also be obtained by other means. Further, the input of the user may further include the spectrum signal in a frequency domain generated by performing the Fourier transform on the spectrum signal in the time domain.

In 620, one or more operators may be determined by the operator unit 420. The operators may be selected by the user or may also be selected by the user from various options given by the compound editing and interaction system(s) 130. Alternatively, the compound editing and interaction system(s) 130 may give a default operator to be calculated and remind the user that the current option is the default operator.

In 630, whether a current edited composite meets a requirement may be determined. If the current edited composite does not meet the user's requirement, such as a false input, or the corresponding composite needs to be determined based on the diagnostic requirements of an object, or an anomaly needs to be handled, the process may proceed to perform operation 610 or operation 620 to reselect the metabolites and/or the operators to be edited. The anomaly may include that the edited composite has no physical or chemical significance. For example, the edited composite may not exist, or the metabolite with the concentration of zero may be as a denominator, resulting the anomaly in mathematical calculations.

If the current edited composite meets the user's requirement, the process may proceed to perform operation 640 to add the edited composite to the display module 330. The edited composite may be stored in a composite collection unit, such as in the database(s) 140.

In 650, the concentration of the edited composite may be determined by the calculation unit 320. The calculated concentration of the composite may include the spatial distribution of the concentration of the composite or the change of the spatial distribution of the concentration of the composite. For example, a single slice and multi-voxel spectrum with size 18*14 (i.e., CSI data) may include 252 voxels. The concentration matrix with size of 18*14 may be generated by the calculation module 320. The positions of the CSI data displayed on the anatomical image and the size of the FOV and the VOI may be determined based on the spatial positional relationship between the CSI data and the anatomical image. And the concentration matrix with size of 18*14 may be interpolated to generate an interpolated concentration matrix with the same size of the FOV. Then the spatial distribution of the concentration of the edited and generated composite in the FOV in the anatomical image may be obtained.

In 660, the calculation result may be displayed. The spatial distribution of the concentration of the composite in the FOV in the anatomical image calculated by operation 650 may be displayed in fusion with the anatomical image, and then the distribution of the concentration of the composite may be displayed in the VOI. In some embodiments, the change of the distribution of the concentration with time may also be displayed. For example, the change of the distribution of the concentration of the edited composite in the VOI with time may be obtained by performing the above operations on the anatomical images taken at different times in time order.

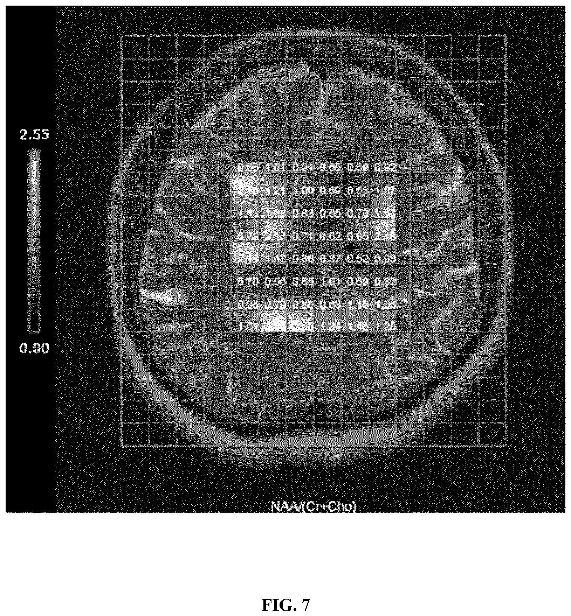

FIG. 7 illustrates an image of the spatial distribution of the concentration of composite NAA/(Cr+Cho) obtained according to process in FIG. 6. The distribution of the value of the concentration of composite NAA/(Cr+Cho) in each voxel may be displayed on an image simultaneously. A region with the highest PPM value is roughly located at the lower left part of the image, indicating that this region may include a lesion.

The above description is only a specific embodiment of the present disclosure and should not be considered as the only embodiment. It will be apparent to those skilled in the art that various modifications and changes in form and detail may be made without departing from the principles and structure of the present disclosure. However, such amendments and changes are still within the scope of the claims of the present disclosure.

The basic concept has been described above, and it is obvious to those skilled in the art that the disclosure of the invention is merely exemplary and does not constitute a limitation of the present disclosure. Various modifications, improvements and improvements to the present disclosure may be made by those skilled in the art, although not explicitly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment", "one embodiment", or "an alternative embodiment" in various parts of this specification are not necessarily all referring to the same embodiment. In addition, certain features, structures, or features of one or more embodiments of the present disclosure may be combined as appropriate.

Moreover, those skilled in the art will appreciate that aspects of the present disclosure may be illustrated and described by a number of patentable categories or conditions, including any new and useful combinations of processes, machines, products or substances, or any new and useful improvements to them. Accordingly, various aspects of the present disclosure may be performed entirely by hardware, may be performed entirely by softwares (including firmware, resident softwares, microcode, etc.), or may be performed by a combination of the hardware and the softwares. The above hardware or softwares may be referred to as "data block", "module", "engine", "unit", "component", or "system". In addition, aspects of the present disclosure may be embodied as a computer product located on one or more computer-readable media, including a computer-readable program code.

The computer-readable signal medium may include a propagated data signal with a computer-readable program code embodied therein, for example, in a baseband or as part of a carrier wave. The propagated signal may have a variety of manifestations, including electromagnetic forms, optical forms, or the like, or a suitable combination thereof. The computer-readable signal medium may be any computer-readable medium that is not a computer-readable storage medium and that may communicate, propagate, or transport a program for use by or in connection with a command execution system, a apparatus, or a device. The program code located on the computer-readable signal medium may be propagated by any suitable medium, including a radio, a cable, a fiber optic cable, a RF, a similar medium, or any combination of the medium.

The computer program code for carrying out the operations for aspects of the present disclosure may be written in any combination of one or more programming languages, including an object oriented programming language, such as Java, Scala, Smalltalk, Eiffel, JADE, Emerald, C++, C#, VB. NET, Python, or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure method does not mean that the present disclosure object requires more features than the features mentioned in the claims. Rather, claim subject matter lie in less than all features of a single foregoing disclosed embodiment.

In some embodiments, the numbers expressing quantities or properties used to describe and claim certain embodiments of the application are to be understood as being modified in some instances by the term "about", "approximate", or "substantially". Unless otherwise stated, "about", "approximate", or "substantially" may indicate ±20% variation of the value it describes. Accordingly, in some embodiments, the numerical parameters set forth in the description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

For each of patents, patent applications, patent application publications and other materials, such as articles, books, commands, publications, documents, articles, etc., cited in this application are hereby incorporated by a reference in their entirety. Application history documents that are inconsistent or conflicting with the contents of the present application are excluded, and documents (currently or later attached to the present application) that limit the widest range of the scope of the present application are also excluded. It is to be noted that if the description, definition, and/or terminology used in the appended application of the present application is inconsistent or conflicting with the contents described in this application, the description, definition and/or terminology may be subject to the present application.

At last, it should be understood that the embodiments described in the present disclosure are merely illustrative of the principles of the embodiments of the present disclosure. Other modifications that may be employed may be within the scope of the application. Thus, by way of example, but not of limitation, alternative configurations of the embodiments of the application may be utilized in accordance with the teachings herein. Accordingly, embodiments of the present disclosure are not limited to the embodiments that are expressly introduced and described herein.

## Claims

1. A system for magnetic resonance spectroscopy, comprising:
a computer-readable storage medium configured to store executable modules including:
an editing module configured to select, based on an input of a user, at least two metabolites and one or more operators to generate a composite;
a calculation module configured to calculate a concentration of the composite; and
a display module configured to display to the user a calculation result including the concentration of the composite; and
a processor configured to execute the executable modules stored in the computer-readable storage medium.

2. The system of claim 1, wherein the editing modules includes a metabolite list unit configured to provide a plurality of metabolites for selection.

3. The system of claim 2, wherein the editing modules further includes an operator unit configured to provide a plurality of operators for selection.

4. The system of claim 3, wherein the editing modules further includes a preview unit configured to display one or more compounds and the one or more operators.

5. The system of claim 1, wherein the calculation module is configured to receive the composite from the editing module and calculate the concentration of the composite.

6. The system of claim 5, wherein the calculation module is configured to analyze the composite using a reverse polish notation algorithm to obtain the at least two metabolites and the one or more operators, and calculate the concentration of the composite based on a concentration of each of the at least two metabolites and the one or more operators.

7. The system of claim 1, wherein the display module is configured to display a spatial distribution of the concentration of the composite in an object.

8. A method for analyzing a magnetic resonance spectrum, comprising:
selecting, based on an input of a user, at least two metabolites and one or more operators to generate a composite;
calculating a concentration of the composite; and
displaying a calculation result including the concentration of the composite.

9. The method of claim 8, further including displaying the at least two metabolites and the one or more operators.

10. The method of claim 8, wherein the calculating the concentration of the composite further includes:
analyzing the composite using a reverse polish notation algorithm to obtain the at least two metabolites and the one or more operators; and
calculating the concentration of the composite based on a concentration of each of the at least two metabolites and the one or more operators.

11. The method of claim 10, wherein the displaying the calculation result including the concentration of the composite further includes displaying a spatial distribution of the concentration of the composite in a body of an object.

12. The method of claim 11, wherein the input of the user includes one or more magnetic resonance anatomical images.

13. The method of claim 12, wherein the input of the user includes spectrum signal information of metabolites associated with the composite in a time domain.

14. The method of claim 13, wherein the one or more magnetic resonance anatomical images and the spectrum signal information are information of the object obtained simultaneously.

15. A computer-readable storage medium configured to store an executable program to perform following operations, comprising:
selecting, based on an input of a user, at least two metabolites and one or more operators to generate a composite;
calculating a concentration of the composite; and
displaying to the user a calculation result including the concentration of the composite; and
a processor configured to execute the executable program stored in the computer-readable storage medium.

16. The storage medium of claim 15, wherein the executable program further performs an operation including displaying the at least two metabolites and the one or more operators.

17. The storage medium of claim 15, wherein the executable program further performs the following operations including:
calculating the concentration of the composite including analyzing the composite using a reverse polish notation algorithm to obtain the at least two metabolites and the one or more operators; and
calculating the concentration of the composite based on a concentration of each of the at least two metabolites and the one or more operators.

18. The storage medium of claim 17, wherein the executable program further performs the following operations:
displaying the calculation result including the concentration of the composite further including displaying a spatial distribution of the concentration of the composite in an object.

19. The storage medium of claim 18, wherein the executable program further performs the following operations:
the input of the user including one or more magnetic resonance anatomical images.

20. The storage medium according to claim 19, wherein the executable program further performs the following operations:
the input of the user including spectrum signal information of metabolites related to the composite in a time domain.
